# EUROPÄISCHE PATENTSCHRIFT

(11) **EP 1 339 332 B1**
(45) Veröffentlichungstag und Bekanntmachung des Hinweises auf die Patenterteilung: **14.07.2004**
(21) Anmeldenummer: 01999322.9
(22) Anmeldetag: 04.12.2001
(51) Int. Cl.: A61B 17/28

(54) **MEDIZINISCHES INSTRUMENT**
MEDICAL INSTRUMENT
INSTRUMENT MEDICAL

(30) Priorität: 07.12.2000 DE 10060769
(43) Veröffentlichungstag der Anmeldung: 03.09.2003
(73) Patentinhaber: Karl Storz GmbH & Co. KG, 78532 Tuttlingen (DE)
(72) Erfinder: LANG, Dieter, 96342 Stockheim (DE); HOPF, Thomas, 96342 Stockheim (DE)
(74) Vertreter: Hofmeister, Frank Horst
(86) Internationale Anmeldenummer: PCT/EP2001/014129
(87) Internationale Veröffentlichungsnummer: WO 2002/045599

(56) Entgegenhaltungen:
- DE-A- 19 534 618
- DE-A- 19 702 079
- DE-U- 9 320 450
- US-A- 5 826 776

## Beschreibung

Die Erfindung betrifft ein medizinisches Instrument mit einem Schaft, an dessen proximalem Ende eine aus mindestens zwei Griffteilen bestehende Handhabe angeordnet ist und an dessen distalem Ende ein aus mindestens zwei Maulteilen bestehendes Werkzeug angeordnet ist, wobei mindestens ein Maulteil des Werkzeugs zum Öffnen und Schließen über jeweils ein verschwenkbar ausgebildetes Griffteil der Handhabe gegenüber dem mindestens einen anderen Maulteil des Werkzeugs verstellbar ist und jedes verstellbare Maulteil und das entsprechende, zum Verstellen des Maulteils dienende Griffteil der Handhabe über eine Zug-/Schubstange miteinander verbunden sind, wobei jedes verstellbare Maulteil über einen Ausgleichshebel mit der jeweiligen Zug-/ Schubstange verbunden ist.

Gattungsgemäße Instrumente lenken das bewegliche Maulteil bzw. die beweglichen Maulteile über einen Hebelmechanismus an. Der Winkel zwischen der Instrumentenachse und der Linie durch den Drehpunkt des Maulteils und des Angriffspunkts am Hebel wird dabei möglichst groß gewählt. Beim Öffnen oder Schließen der Maulteile kommt es aber zwangsläufig zu einer Verkleinerung des Winkels, was die Kraftübertragung deutlich verschlechtert. Besonders bei medizinischen Stanzen für Gewebe ist es unbekannt, in welcher Winkelstellung der Maulteile besonders gut die Kraft übertragen werden muß, da es sich um unterschiedlich dicke Gewebe handeln kann.

Ein gattungsgemäßes chirurgisches Instrument ist beispielsweise aus der US 5 826 776 A bekannt. Dieses bekannte Instrument weist ein aus zwei Maulteilen bestehendes Werkzeug auf, das über eine von der Handhabe betätigten Zug-/Schubstange geöffnet und geschlossen werden kann. Wie aus den Abbildungen Fig. 34A und 34B ersichtlich, ist bei diesem bekannten Instrument das verstellbare Maulteil über einen Ausgleichshebel mit der Zug-/Schubstange verbunden, der verschiebbar in einer im verstellbaren Maulteil ausgebildeten Führungsnut gelagert ist. Dieser Ausgleichshebel ist zusätzlich zur Lagerung an der Zug-/Schubstange auch noch am starren Maulteil gelagert, so daß ein direktes und nahezu spielfreies Führen des Ausgleichshebels und somit des verstellbaren Maulteils über die Zug-/Schubstange und somit die Handhabe bei dieser Ausgestaltungsform nicht möglich ist. Aufgrund der festen Lagerung des Ausgleichshebels am starren Maulteil dient die Zug-/Schubstange nur dazu, den Ausgleichshebel vor und zurück zu kippen, wie dies die Abbildungen zeigen. Ein Führen mit dauerhaftem Kontakt zum verstellbaren Maulteil ist bei dem bekannten Instrument nicht möglich, so dass das Schnittgefühl zumindest in den Übergangsphasen, in denen der Ausgleichshebel von der Anlage an der einen Seitenwand der Führungsnut zur Anlage an der anderen Seitenwand wechselt, deutlich beeinträchtigt ist.

Bei einer weiteren, aus der DE 199 06 360 A1 bekannten Konstruktion ist zwischen der Betätigungsstange und dem verschwenkbaren Maulteil ein Zugglied angeordnet, über das das Maulteil wieder in die geschlossene Stellung überführbar ist.

Das Öffnen des Maulteils erfolgt dadurch, daß die Betätigungsstange mit ihrer Spitze gegen eine Druckfläche des Maulteils anläuft und dieses so in die Offenstellung drückt. Um einerseits das Anlaufen der Betätigungsstange gegen die Druckfläche des Maulteils zu ermöglichen und andererseits das Schließen des Maulteils über das Zugglied zu ermöglichen, weist die Lagerstelle des Zugglieds ander Betätigungsstange eine Langlochführung auf, die das Verlagern der Betätigungsstange ohne Betätigen des Zugglieds erlaubt. Dieses Bewegungsspiel der Betätigungsstange aufgrund der nicht direkten Ankopplung an das Zugglied bewirkt aber für den Operateur einen Zwischenstadium, in dem das Schnittgefühl beeiträchtigt wird, da keine unmittelbare Kopplung zwischen dem Maulteil und der mit der Betätigungsstange verbundenen Handhabe besteht.

Ein weiteres medizinisches Instrument ist aus der US 4,712,545 A bekannt. Bei diesem bekannten chirurgischen Instrument wird das bewegliche Maulteil auf einer Bogenbahn im starren Teil des Schaftes und auf einer weiteren Bogenbahn zu einer Schubstange geführt. Diese Ausgestaltung führt zu gedachten Drehpunkten, die teilweise außerhalb des Instrumentenschafts liegen. Durch diese Maßnahme wird allerdings nur der Hebelarm vergrößert - ähnliches kann auch durch Maßnahmen an der Handhabe realisiert werden - und damit in jeder Position mehr Kraft übertragen. Je mehr aber das Kräfteparalletogramm gestreckt ist, um so ungünstiger wird das Hebelverhältnis, so daß im Extremfall auch bei großem Hebelarm keine Kraft mehr übertragen werden kann.

Die weiterhin aus dem Stand der Technik bekannte US 6,019,780 A offenbart auch ein medizinisches Instrument, bei dem der Drehpunkt über eine Bahnführung nach außen verlagert ist. Ebenso wie bei der US 4,712,545 A bewirkt auch bei diesem bekannten Instrument die Bahnführung eine zusätzliche Reibung, die das Schnittgefühl des Operateurs beeinträchtigt.

Ausgehend von diesem Stand der Technik liegt der Erfindung die **Aufgabe** zugrunde, ein medizinisches Instrument der eingangs genannten Art so zu verbessern, daß dieses eine bestmögliche Kraftübertragung bei einem guten Schnittgefühl für den Operateur gewährleistet.

Die **Lösung** dieser Aufgabenstellung ist erfindungsgemäß dadurch gekennzeichnet, daß der Ausgleichshebel mit einem freien Ende ausschließlich an der Zug-/ Schubstange gelagert ist und an dieser schwenkbar festgelegt ist, und mit dem anderen freien Ende verschiebbar in einer im verstellbaren Maulteil ausgebildeten Führungsnut gelagert ist.

Aufgrund dieser Ausgestaltung ist sichergestellt, daß für die bestmögliche Kraftübertragung immer ein gleichbleibendes Hebelverhältnis zwischen der Zug-/ Schubstange und dem verstellbaren Maulteil vorliegt, da der Ausgleichshebel und die Zug-/ Schubstange vorteilhafterweise verschwenkbar, aber ansonsten fest miteinander verbunden sind. Diese feste Verbindung stellt zusammen mit der verschiebbaren Führung des Ausgleichshebels im verstellbaren Maulteil sicher, daß die Zug-/ Schubstange bei der Betätigung des verschwenkbaren Griffteils keine die Reibung erhöhende Pendelbewegung ausführt. Darüber hinaus ermöglicht die flächige plane Anlage des Ausgleichshebels am Maulteil in der Führungsnut eine direkte und feinfühlige Kraftübertragung auf das verstellbare Maulteil.

Im Gegensatz zum Stand der Technik bewirkt die erfindungsgemäße Lagerung des Ausgleichshebels ausschließlich an der Zug-/Schubstange, daß der Operateur immer ein direktes Gefühl für die Bewegung und Führung des verstellbaren und spielfrei geführten Maulteils hat.

Eine nahezu spielfreie Führung des Ausgleichshebels kann weiterhin dadurch erzielt werden, daß der Ausgleichshebel im wesentlichen formschlüssig in der Führungsnut geführt ist.

Zur Verbesserung der Reinigbarkeit und zur Montage- und Reparaturerleichterung des medizinischen Instruments sind gemäß einer bevorzugten Ausführungsform der Erfindung der Ausgleichshebel und die Zug-/ Schubstange als Einheit aus dem Schaft entnehmbar.

Gemäß einer ersten Ausführungsform der Erfindung ist der Ausgleichshebel als als im Querschnitt im wesentlichen kreisfömiger Bolzen ausgebildet ist.

Gemäß einer alternativen Ausführungsform der Erfindung vorgeschlagen, daß der Ausgleichshebel als im Querschnitt unrunder Bolzen ausgebildet ist.

Schließlich wird mit der Erfindung vorgeschlagen, daß ein Verbindungspunkt zwischen dem Ausgleichshebel und der Zug-/ Schubstange oberhalb eines Drehpunkts des verstellbaren Maulteils angeordnet ist.

Weitere Merkmale und Vorteile der Erfindung ergeben sich aus der nachfolgenden Beschreibung der zugehörigen Zeichnung, in der ein Ausführungsbeispiel eines erfindungsgemäßen medizinischen Instruments nur beispielhaft schematisch dargestellt ist. In der Zeichnung zeigt:
- Fig. 1: eine Seitenansicht eines erfindungsgemäßen medizinischen Instruments, die Maulteile im geschlossenen Zustand darstellend;
- Fig. 2a: eine vergrößerte und teilweise geschnittene Darstellung des Details IIa gemäß Fig. 1 und
- Fig. 2b: eine Darstellung gemäß Fig. 2a, jedoch die Maulteile im geöffneten Zustand darstellend.

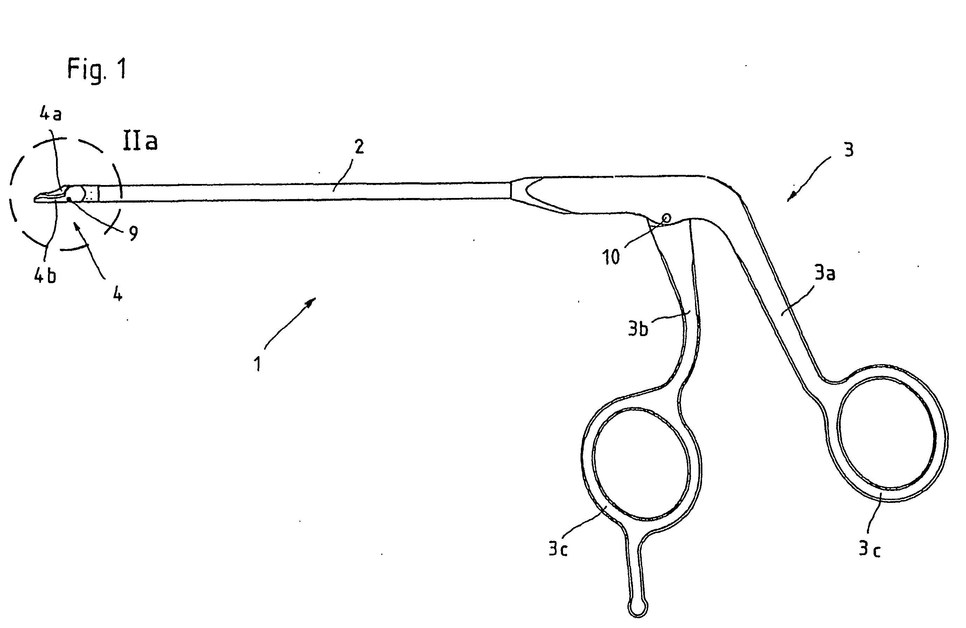

Die Abbildung Fig. 1 zeigt eine Seitenansicht eines medizinischen Instruments 1, dessen Kraftübertragungsmechanismus vielseitig verwendet werden kann, wie beispielsweise für Stanzen, Scheren, Nadelhalter, Faßinstrumente und dergleichen.

Das medizinische Instrument 1 besteht im wesentlichen aus einem Schaft 2, an dessen proximalem Ende eine Handhabe 3 angeordnet ist, die aus einem starren Griffteil 3a und einem gegenüber dem starren Griffteil 3a verschwenkbaren Griffteil 3b besteht. Am distalen Ende des Schaftes 2 ist ein Werkzeug 4 angeordnet, welches beim dargestellten Ausführungsbeispiel ein verstellbares Maulteil 4a und ein starr mit dem Schaft 2 verbundenes Maulteil 4b aufweist.

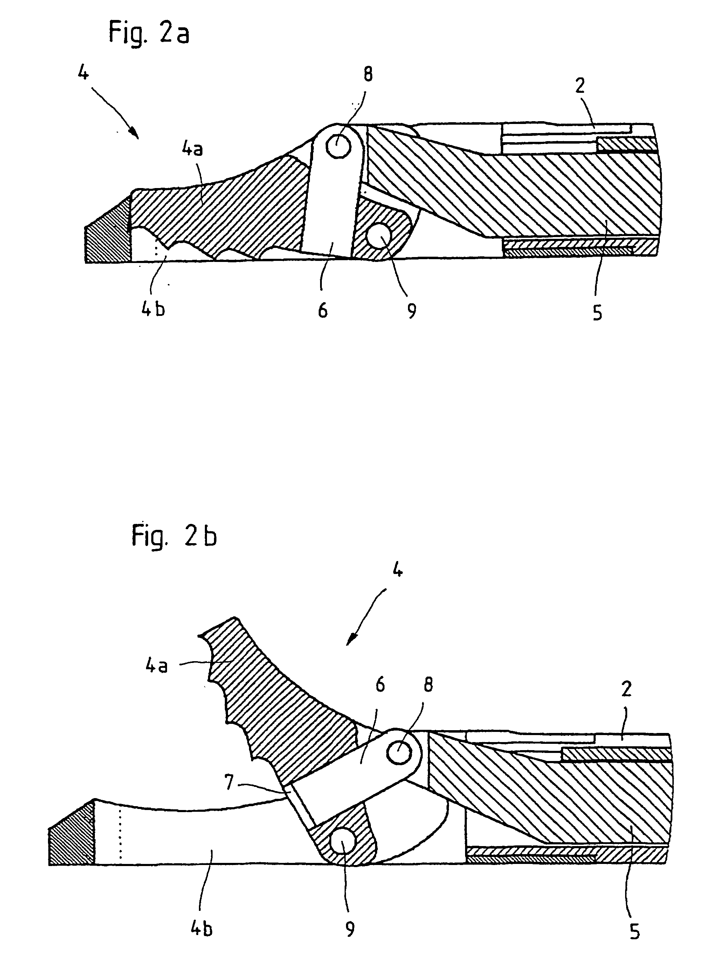

Wie aus den Detailansichten gemäß Fig. 2a und 2b ersichtlich, sind das verstellbare Maulteil 4a des Werkzeugs 4 und das verschwenkbare Griffteil 3b der Handhabe 3 über eine Zug- / Schubstange 5 miteinander so verbunden, daß durch das Verschwenken des Griffteils 3b das verstellbare Maulteil 4a von der geschlossenen Stellung (Fig. 1 und 2a) in die offene Stellung (Fig. 2b) bzw. umgekehrt überführt werden kann. Bei dem dargestellten medizinischen Instrument 1 ist der Schaft 2 zur Aufname der Zug-/ Schubstange 5 hohl ausgebildet.

Um eine möglichst gute Kraftübertragung zwischen der Handhabe 3 und dem verstellbaren Maulteil 4a des Werkzeugs 4 zu erzielen, sind, wie insbesondere aus Fig. 2b ersichtlich, die Zug-/ Schubstange 5 und das verstellbare Maulteil 4a über einen Ausgleichshebel 6 miteinander verbunden, wobei der Ausgleichshebel 6 verschiebbar in einer im verstellbaren Maulteil 4a ausgebildeten Führungsnut 7 gelagert ist und ein Verbindungspunkt 8 zwischen dem Ausgleichshebel 6 und der Zug-/Schubstange 5 oberhalb eines Drehpunkts 9 des verstellbaren Maulteils 4a angeordnet, ist.

Die Verbindung von Zug-/ Schubstange 5 und verstellbarem Maulteil 4a über den verschiebbar im Maulteil 4a geführten Ausgleichshebel 6 ermöglicht ein gleichbleibendes Hebelverhältnis zwischen den beiden Bauteilen 5 und 4a ohne die Gefahr einer Pendelbewegung der Zug-/ Schubstange 5. Aufgrund dieses gleichbleibenden Hebelverhältnisses hat der Operateur immer ein gutes und gleichmäßiges Schnittgefühl.

Zum sicheren Ergreifen der Griffteile 3a, 3b der Handhabe 3 weisen diese an ihren freien Enden Fingerösen 3c auf. Beim dargestellten Ausführungsbeispiel ist das Griffteil 3b um eine Schwenkachse 10 gegenüber dem anderen, starren Griffteil 3a verschwenkbar. Der Verschwenkweg der beiden Griffteile 3a, 3b zueinander läßt sich durch eine nicht dargestellte Übersetzung verkürzen. Ebenso ist es möglich, beide Griffteile 3a, 3b der Handhabe 3 als verschwenkbare Griffteile auszubilden. Durch die Kopplung des verschwenkbaren Griffteils 3b über die Zug-/ Schubstange 5 und den Ausgleichshebel 6 mit dem verstellbaren Maulteil 4a läßt sich das Werkzeug 4 beim Betätigen der Handhabe 3 öffnen und schließen.

Das Reinigen und Reparieren des medizinischen Instruments 1 wird dadurch erleichtert, daß die Zug-/ Schubstange 5 und der Ausgleichshebel 6 als komplette Einheit aus dem hohlen Schaft 2 entnehmbar sind.

Insgesamt zeichnet sich das dargestellte medizinische Instrument 1 dadurch aus, daß es eine gleichmäßig starke Kraftübertragung unabhängig von der Öffnungsstellung der Maulteile gewährleistet und es möglich ist, diese Kraft dosiert einzusetzen, so daß nach dem Durchtrennen harten Gewebes kein unkontrolliertes Durchschlagen der kraftbeanspruchten Maulteile erfolgt.

### Bezugszeichenliste

- 1: medizinisches Instrument
- 2: Schaft
- 3: Handhabe
- 3a: starres Griffteil
- 3b: verschwenkbares Griffteil
- 3c: Fingeröse
- 4: Werkzeug
- 4a: verstellbares Maulteil
- 4b: starres Maulteil
- 5: Zug- / Schubstange
- 6: Ausgleichshebel
- 7: Führungsnut
- 8: Verbindungspunkt
- 9: Drehpunkt
- 10: Schwenkachse

## Patentansprüche

1. Medizinisches Instrument mit einem Schaft (2), an dessen proximalem Ende eine aus mindestens zwei Griffteilen (3a, 3b) bestehende Handhabe (3) angeordnet ist und an dessen distalem Ende ein aus mindestens zwei Maulteilen (4a, 4b) bestehendes Werkzeug (4) angeordnet ist, wobei mindestens ein Maulteil (4a) des Werkzeugs (4) zum Öffnen und Schließen über jeweils ein verschwenkbar ausgebildetes Griffteil (3b) der Handhabe (3) gegenüber dem mindestens einen anderen Maulteil (4b) des Werkzeugs (4) verstellbar ist und jedes verstellbare Maulteil (4a) und das entsprechende, zum Verstellen des Maulteils (4a) dienende Griffteil (3b) der Handhabe (3) über eine Zug-/ Schubstange (5) miteinander verbunden sind, wobei jedes verstellbare Maulteil (4a) über einen Ausgleichshebel (6) mit der jeweiligen Zug-/ Schubstange (5) verbunden ist,
**dadurch gekennzeichnet,**
**daß** der Ausgleichshebel (6) mit einem freien Ende ausschließlich an der Zug-/ Schubstange (5) gelagert ist und an dieser schwenkbar festgelegt ist, und mit dem anderen freien Ende verschiebbar in einer im verstellbaren Maulteil (4a) ausgebildeten Führungsnut (7) gelagert ist

2. Medizinisches Instrument nach Anspruch 1, **dadurch gekennzeichnet, daß** der Ausgleichshebel (6) im wesentlichen formschlüssig in der Führungsnut (7) geführt ist.

3. Medizinisches Instrument nach Anspruch 1 oder 2, **dadurch gekennzeichnet, daß** der Ausgleichshebel (6) und die Zug-/ Schubstange (5) als Einheit aus dem Schaft (2) entnehmbar sind.

4. Medizinisches Instrument nach mindestens einem der Ansprüche 1 bis 3, **dadurch gekennzeichnet, daß** der Ausgleichshebel (6) als im Querschnitt im wesentlichen kreisfömiger Bolzen ausgebildet ist.

5. Medizinisches Instrument nach mindestens einem der Ansprüche 1 bis 3, **dadurch gekennzeichnet, daß** der Ausgleichshebel (6) als im Querschnitt unrunder Bolzen ausgebildet ist.

6. Medizinisches Instrument nach mindestens einem der Ansprüche 1 bis 5, **dadurch gekennzeichnet, daß** ein Verbindungspunkt (8) zwischen dem Ausgleichshebel (6) und der Zug-/ Schubstange (5) oberhalb eines Drehpunkts (9) des verstellbaren Maulteils (4a) angeordnet ist.

## Claims

1. Medical instrument with a shaft (2) at whose proximal end there is a handle (3) consisting of at least two grip parts (3a, 3b), and at whose distal end there is a tool (4) consisting of at least two jaw parts (4a, 4b), at least one jaw part (4a) of the tool (4) being adjustable relative to the at least one other jaw part (4b) of the tool (4) for the purpose of opening and closing via a respective pivotable grip part (3b) of the handle (3), and each adjustable jaw part (4a) and the corresponding grip part (3b) of the handle (3) serving for adjustment of the jaw part (4a) being connected to one another via a pull/push rod (5), each adjustable jaw part (4a) being connected to the respective pull/push rod (5) via a compensation lever (6), **characterized in that** the compensation lever (6) is mounted with a free end exclusively on the pull/push rod (5) and is fixed pivotably thereon, and the other free end is mounted displaceably in a guide groove (7) formed in the adjustable jaw part (4a).

2. Medical instrument according to Claim 1, **characterized in that** the compensation lever (6) is guided substantially with a form fit in the guide groove (7).

3. Medical instrument according to Claim 1 or 2, **characterized in that** the compensation lever (6) and the pull/push rod (5) can be removed as a unit from the shaft (2).

4. Medical instrument according to at least one of Claims 1 to 3, **characterized in that** the compensation lever (6) is designed as a bolt which has a substantially circular cross section.

5. Medical instrument according to at least one of Claims 1 to 3, **characterized in that** the compensation lever (6) is designed as a bolt which has a non-circular cross section.

6. Medical instrument according to at least one of Claims 1 to 5, **characterized in that** a connection point (8) between the compensation lever (6) and the pull/push rod (5) is arranged above a pivot point (9) of the adjustable jaw part (4a).

## Revendications

1. Instrument médical, comportant une tige (2), sur l'extrémité proximale de laquelle est montée une poignée (3) constituée par au moins deux éléments de préhension (3a, 3b) et sur l'extrémité distale de laquelle est disposé un outil (4) constitué par au moins deux éléments de mâchoire (4a, 4b), dans lequel au moins un élément de mâchoire (4a) est réglable par rapport à le au moins un autre élément de mâchoire (4b) de l'outil (4) pour la fermeture et l'ouverture, par l'intermédiaire respectivement d'un élément de préhension (3b), agencé de manière à être pivotant, de la poignée (3), et chaque élément de mâchoire réglable (4a) et l'élément de préhension correspondant (3b), utilisé pour le réglage de l'élément de mâchoire (3a), de la poignée (3) sont reliés entre eux par l'intermédiaire d'une tige de traction / poussée (5), chaque élément de mâchoire (4a) étant relié par l'intermédiaire d'un levier de compensation (6) à la tige respective de traction / poussée (5),
**caractérisé en ce**
**que** le levier de compensation (6) est monté, au moyen de son extrémité libre, exclusivement sur la tige de traction / poussée (5) et est fixé de manière à pouvoir pivoter sur cette tige et est monté, par son autre extrémité libre, de manière à être déplaçable dans une rainure de guidage (7) formée dans l'élément de mâchoire réglage (4a).

2. Instrument médical selon la revendication 1, **caractérisé en ce que** le levier de compensation (6) est guidé essentiellement selon une liaison par formes complémentaires dans la rainure de guidage (7).

3. Instrument médical selon la revendication 1 ou 2, **caractérisé en ce que** le levier de compensation (6) et la tige de traction / poussée (5) peuvent être retirés de la tige (2), sous la forme d'une unité.

4. Instrument médical selon au moins l'une des revendications 1 à 3, **caractérisé en ce que** le levier de compensation (6) est agencé sous la forme d'un goujon essentiellement circulaire en coupe transversale.

5. Instrument médical selon au moins l'une des revendications 1 à 3, **caractérisé en ce que** le levier de compensation (6) est agencé sous la forme d'un goujon non circulaire en coupe transversale.

6. Instrument médical selon au moins l'une des revendications 1 à 5, **caractérisé en ce qu'**un point de jonction (8) entre le levier de compensation (6) et la tige de traction / poussée (5) est disposé au-dessus d'un centre de rotation (9) de l'élément de mâchoire réglage (4a).
